# EUROPEAN PATENT APPLICATION

(11) **EP 1 142 565 A1**
(43) Date of publication of application: **10.10.2001**
(21) Application number: 00969849.9
(22) Date of filing: 18.10.2000
(51) Int. Cl.: A61K 9/08, A61K 47/10, A61K 47/36, A61K 47/38, A61K 31/573

(54) **AQUEOUS MEDICINAL COMPOSITIONS**

(30) Priority: 20.10.1999 JP 29818799
(71) Applicant: TEIJIN LIMITED, Osaka-shi Osaka 541-0054 (JP)
(72) Inventor: NAGANO, Atsuhiro Teijin Ltd., Tokyo Res. Ctr., Hino-shi Tokyo 191-0065 (JP); NISHIBE, Yoshihisa Teijin Ltd., Iwakuni Res. Ctr., Iwakuni-shi Yamaguchi 740-0014 (JP); TAKANASHI, Kazuya Teijin Limited, Tokyo Res. Ctr., Hino-shi Tokyo 191-0065 (JP)
(74) Representative: Hallybone, Huw George
(86) International application number: JP0007228
(87) International publication number: WO0128517

(57) **Abstract**

An aqueous pharmaceutical composition containing one or more water-insoluble and/or low water-soluble medicament except ciclesonide and hydroxypropylmethylcellulose, wherein the one or more water-insoluble and/or low water-soluble medicament except ciclesonide are dispersed as solid particles in the aqueous pharmaceutical composition.

## Description

### Technical Field

This invention relates to an aqueous pharmaceutical composition for use in drug therapy which is an aqueous pharmaceutical composition containing one or more water-insoluble and/or low water-soluble medicament except ciclesonide and hydroxypropylmethylcellulose and is characterized in that the one or more water-insoluble and/or low water-soluble medicament are dispersed as solid particles in an aqueous medium. In detail, the invention relates to the aqueous pharmaceutical composition having more excellent medicament dispersibility in production than that of a conventional aqueous pharmaceutical composition.

### Background Art

An aqueous pharmaceutical composition containing a water-insoluble or low water-soluble medicament as solid particles in an aqueous medium is recognized as one of useful dosage forms for reasons that 1. complete dissolution of the low water-soluble medicament is not required, 2. the low water-soluble medicament can be directly applied to disease sites of local diseases of nasal mucosa, eyes, epidermis, etc. by spray, etc. 3. the low water-soluble medicament is more easily swallowed than tablet, granule, etc.

Among the water-insoluble and/or low water-soluble medicament, some water-insoluble and/or low water-soluble medicament are hardly wettable and are liable to aggregate in the aqueous medium. Consequently, in order to disperse the water-insoluble and/or low water-soluble medicament in a stable state in an aqueous medium, methods such as addition of a wetting agent, e.g. polysorbate 80, etc. vigorous stirring during production, etc. have been carried out.

Improvement in medicament dispersibility of an aqueous pharmaceutical composition containing a water-insoluble and/or low water-soluble medicament as solid particles in an aqueous medium by addition of a cellulose-based polymer has been disclosed by the patent of Morishima (WO99/37286 specification), et al. However, the patent relates to redispersion of the medicament settled during preservation and is fundamentally different from this invention related to transfer of the medicament to foam occurred by vigorous stirring during the production and adsorption of the medicament on the wall surface of a production apparatus. Additionally the concentration of the cellulose-based polymer of the patent of Morishima, et al. is 0.0001-0.003%, methylcellulose can be substituted as the cellulose-based polymer besides hydroxypropylmethylcellulose and addition of a nonionic surfactant is required. This invention having the optimum value of hydroxypropylmethylcellulose concentration of not less than 0.01% w/w and not more than 0.5% w/w and not requiring the surfactant is not readily deduced from the patent.

In the production process of the aqueous medium composition, high shear stress is required so as to disperse the water-insoluble and/or low water-soluble medicament except ciclesonide and vigorous stirring of the aqueous pharmaceutical composition is needed. Since the medicament is transferred to foams occurring in the stirring and the medicament concentration at the upper part of the aqueous pharmaceutical composition is made higher than that at the lower part of the pharmaceutical composition, the medicament concentration of the produced aqueous pharmaceutical composition causes a variation. Further, recovery of the medicament is dropped by adhesion of the medicament to the wall surface, etc. of the production apparatus. The phenomenon becomes prominent with the rise of lipophilicity of the medicament.

The variation of the medicament concentration and the adhesion of the medicament to the production apparatus are hardly improved by the addition of polysorbate 80, etc. being the wetting agent which has been conventionally used, and, on the contrary, the addition of the wetting agent results in increase in occurrence of foam and further enlargement of the variation of the medicament concentration.

Therefore, development of an aqueous pharmaceutical composition capable of avoiding the variation of the medicament concentration during production and reduction in recovery of the medicament is keenly expected.

Namely, the purpose of this invention is to provide the aqueous pharmaceutical composition for avoiding the variation of the medicament concentration during the production and reduction in the recovery of the medicament.

As the result of the keen examination of this inventor, et al. so as to solve the above-mentioned problems, the inventor, et al. found that the aqueous pharmaceutical composition avoiding the variation of the medicament concentration during the production and reduction in recovery of the medicament more than a conventional aqueous pharmaceutical composition can be provided and reached this invention by preparing the aqueous pharmaceutical composition containing the water-insoluble and/or low water-soluble medicament except ciclesonide and hydroxypropylmethylcellulose.

Namely, this invention is the aqueous pharmaceutical composition containing one or more water-insoluble and/or low water-soluble medicament except ciclesonide and hydroxypropylmethylcellulose and is the aqueous pharmaceutical composition characterized in that the one or more water-insoluble and/or low water-soluble medicament are dispersed as solid particles in the aqueous dispersion.

### Disclosure of Invention

Namely, this invention is the aqueous pharmaceutical composition containing one or more water-insoluble and/or low water-soluble medicament except ciclesonide and hydroxypropylmethylcellulose and is the aqueous pharmaceutical composition characterized in that the one or more water-insoluble and/or low water-soluble medicament except ciclesonide are dispersed as solid particles in the aqueous dispersion.

### Best Mode for Carrying Out the Invention

Hereinafter this invention is explained in detail.

The aqueous pharmaceutical composition of this invention contains one or more water-insoluble and/or low water-soluble medicament (hereinafter may be sometimes referred as "medicament" of this invention) except ciclesonide and hydroxypropylmethylcellulose.

Any medicament are applicable as the medicament of this invention as long as they are except ciclesonide and are water-insoluble or low water-soluble. For example, a medicament for sedative hypnotic, a medicament for antianxiety agent, a medicament for anticonvulsant, a medicament for analgesic antipyretic, a medicament for local anesthetic, a medicament for antispasmodic, a medicament for cardiac stimulant, a medicament for diuretic, a medicament for vasoconstrictor, a medicament for bronchodilator, a medicament for peptic ulcer, a medicament for analgesic, a medicament for hormone preparation, a medicament for antidote, vaccines, antibiotic, a medicament for chemotherpeutic, a medicament for anti-Parkinson agent, a medicament for psychoneurotic, a medicament for muscle relaxant, a medicament for antiarrhythmic , a medicament for antihypertensive agent, a medicament for hypolipidemic, a medicament for respiratory stimulant, a medicament for expectorant, a medicament for antiflatuent, vitamins and a medicament for antiallergic may be cited as the medicament. Medicament having relatively high lipophilicity can be especially cited among the medicament and liposoluble vitamins, steroids and prostaglandins can be cited as the concrete examples.

The particle diameters of the medicament of this invention may be any sizes and particle diameters of not smaller than 10 nm and not larger than 100 µm can be cited as a preferable range and especially particle diameters of not smaller than 10 nm and not larger than 10 µm can be cited as preferable range.

A water-insoluble and/or low water-soluble substance, which can be used in this invention, may be any substance and celluloses can be cited as the preferable substances. Microcrystalline cellulose can be especially cited as the substance.

In this invention, the concentration of the water-insoluble and/or low water-soluble substance existing as solid particles in the aqueous medium is preferably not less than 0.3% w/w, more preferably 1% w/w to 10% w/w based on the total amount of a preparation.

In this invention, a water-soluble polymer substance can be added to the composition. To be concrete, propylene glycol alginate, pectin, low methoxyl pectin, guar gum, gum arabic, carrageenan, methylcellulose, carboxymethylcellulose sodium, xanthan gum and hydroxypropylcellulose can be cited as the water-soluble polymer substances and carboxymethylcellulose sodium, polyethylene glycol and hydroxypropylcellulose can be especially cited as the preferable water-soluble polymer substances. Microcrystalline cellulose carmellose sodium is a combination of the water-soluble polymer substance and the water-insoluble substance and is obtained by formulating carboxymethylcellulose sodium with microcrystalline cellulose. When the water-soluble polymers are added to the composition, the concentration of the water-soluble polymers is preferably 1% w/w to 30% w/w based on the water-insoluble and/or low water-soluble substance.

In the aqueous pharmaceutical composition of this invention, the essential condition is to contain hydroxypropylmethylcellulose; any grade of hydroxypropylmethylcellulose may be as the grade of hydroxypropylmethylcellulose and hydroxypropylmethylcellulose 2910 may be cited as the concrete example.

The concentration of hydroxypropylmethylcellulose may be any concentration, preferably from 0.01% w/w to 30% w/w, more preferably from 0.01% w/w to 5% w/w, further preferably from 0.01% w/w to 1% w/w and most preferably from 0.01% w/w to 0.5% w/w.

Although the addition of the wetting agent is not particularly required in this invention, the wetting agent can be added to the composition. Polysorbate 80, glyceryl monostearate, polyoxyl stearate, lauromacrogol, sorbitan oleate, sucrose fatty acid esters, etc. can be cited as the wetting agent.

An osmotic pressure regulating substance (osmotic pressure regulator) can be added to the composition, salts such as sodium chloride, a water-soluble saccharides such as glucose can be cited as the concrete example and the saccharides such as glucose can be especially cited.

The dose of the medicament except ciclesonide used in this invention is an effective therapeutic dose and can be determined according to each medicament, kind and degree of disease, age and weight of patient, etc.

The concentration of the medicament of this invention is preferably 0.01% w/w to 1% w/w, especially preferably 0.05% w/w to 0.5% w/w based on the total amount of the preparation.

A method for producing the aqueous pharmaceutical composition in this invention may be any method for dispersing the water-insoluble and/or low water-soluble substance into the aqueous medium and to be concrete, a method for using a homomixer may be cited as the method.

In order to improve physical properties, appearance or smell, etc. as a preparation, if necessary, a well-known antiseptic, pH controlling agent, preservative, buffer agent, colorant, flavor, etc. may be added to the composition of this invention. Benzalkonium chloride, etc. may be cited as the antiseptic, hydrochloric acid, sodium hydroxide, etc. as the pH controlling agent, ascorbic acid, etc. as the preservative, phosphoric acid and its salt as the buffer agent, artificial dye called Red No. 2, etc. as the colorant and menthol, etc. as the flavor.

In this manner by this invention, the aqueous pharmaceutical composition more avoiding the variation of the medicament concentration during the production and reduction in recovery of the medicament than a conventional aqueous pharmaceutical composition can be provided. The avoidance leads the composition to improvement in quality and reduction in manufacturing cost as well.

Consequently this invention has an extremely high significance in terms of quality and economic effect in the production of the aqueous pharmaceutical composition.

### Examples

Hereinafter this invention is explained by examples but this invention is not limited by the examples.

Hereinafter this invention is explained by the examples.

Budesonide manufactured from Sigma-Aldrich Co., Ltd, beclomethasone dipropionate manufactured from Sicor Co., Ltd, microcrystalline cellulose carmellose sodium being Avicel™ RC-A591NF manufactured from Asahi Chemical Industry Co., Ltd., hydroxypropylmethylcellulose 2910 manufactured from Shin-Etsu Chemical Co., Ltd., polysorbate 80 manufactured from Nippon Surfactant Co., Ltd and polysorbate 60 manufactured from Wako Pure Chemical Industries, Ltd. are used in this invention, respectively. A Robomics™ manufactured from Tokushu Kika Kogyo Co., Ltd was used as the homomixer.

### Example 1

An aqueous pharmaceutical composition comprising the following components was prepared in the scale of 300 ml by a homomixer treatment. The homomixer treatment was carried out at 6,000 rpm for 30 minutes.

| (Composition 1) | |
|---|---|
| Budesonide | 0.1% w/w |
| Microcrystalline cellulose carmellose sodium | 1.7% w/w |
| Hydroxypropylmethylcellulose 2910 | 0.1% w/w |

Immediately after the composition 1 was treated by the homomixer, the aqueous pharmaceutical composition was collected from the upper part and the lower part of an emulsifying tank and a budesonide concentration was determined by HPLC. The value of the budesonide concentration at the upper part of the emulsifying tank was calculated in the case where the budesonide concentration was 100% at the lower part of the emulsifying tank.

Successively, the budesonide concentration of the aqueous pharmaceutical composition recovered from the emulsifying tank was determined by HPLC and the recovery of budesonide was obtained based on the theoretical value of the budesonide concentration calculated form the charged amount of the composition. Table 1 shows these values.

### Comparative example 1

An aqueous pharmaceutical composition comprising the following components was prepared in the scale of 300 ml by the homomixer treatment. The homomixer treatment was carried out at 6,000 rpm for 30 minutes.

| (Composition 2) | |
|---|---|
| Budesonide | 0.1%w/w |
| Microcrystalline cellulose carmellose sodium | 1.7% w/w |
| Hydroxypropylmethylcellulose 2910 | 0.1% w/w |
| Polysorbate 80 | 0.1 % w/w |

Immediately after the composition 2 was treated by the homomixer, the aqueous pharmaceutical composition was collected from the upper part and the lower part of the emulsifying tank and the budesonide concentration was determined by HPLC. The value of the budesonide concentration at the upper part of the emulsifying tank was calculated in the case where the budesonide concentration was 100% at the lower part of the emulsifying tank.

Successively, the budesonide concentration of the aqueous pharmaceutical composition recovered from the emulsifying tank was determined by HPLC and the recovery of budesonide was obtained based on the theoretical value of the budesonide concentration calculated form the charged amount of the composition. Table 1 shows these values.

**Table 1**

| | Preparation | Budesonide concentration immediately after treatment (%) | | Recovery rate (%) |
|---|---|---|---|---|
| | | Upper part of emulsifying tank | Lower part of emulsifying tank | |
| Example 1 | Composition 1 | 99.6 | 100.0 | 102.1 |
| Comparative example 1 | Composition 2 | 109.9 | 100.0 | 97.9 |

### Explanation of table 1

The composition 1 containing 0.1% of hydroxypropylmethylcellulose 2910 had uniform budesonide concentration in the emulsifying tank immediately after the homomixer treatment and approximately 100% recovery. On the other hand, the composition 2 containing 0.1% of polysorbate 80 had budesonide concentration at the upper part of the emulsifying tank immediately after the homomixer treatment about 10% higher than that at the lower part of the emulsifying tank. The recovery of the medicament was reduced by about 2%.

### Example 2

Aqueous pharmaceutical compositions comprising the following components were prepared in the scale of 300 ml by the homomixer treatment. The homomixer treatments were carried out at 6,000 rpm for 30 minutes.

| (Composition 3) | |
|---|---|
| Beclomethasone dipropionate | 0.1% w/w |
| Microcrystalline cellulose carmellose sodium | 1.7% w/w |
| Hydroxypropylmethylcellulose 2910 | 0.01% w/w |

| (Composition 4) | |
|---|---|
| Beclomethasone dipropionate | 0.1% w/w |
| Microcrystalline cellulose carmellose sodium | 1.7% w/w |
| Hydroxypropylmethylcellulose 2910 | 0.1% w/w |

| (Composition 5) | |
|---|---|
| Beclomethasone dipropionate | 0.1% w/w |
| Microcrystalline cellulose carmellose sodium | 1.7% w/w |
| Hydroxypropylmethylcellulose 2910 | 1% w/w |

| (Composition 6) | |
|---|---|
| Beclomethasone dipropionate | 0.1% w/w |
| Microcrystalline cellulose carmellose sodium | 1.7% w/w |
| Hydroxypropylmethylcellulose 2910 | 2.5% w/w |

| (Composition 7) | |
|---|---|
| Beclomethasone dipropionate | 0.1% w/w |
| Microcrystalline cellulose carmellose sodium | 1.7% w/w |
| Hydroxypropylmethylcellulose 2910 | 5% w/w |

Immediately after the compositions 3 to 7 were treated by the homomixer, the aqueous pharmaceutical compositions were collected from the upper part and the lower part of the emulsifying tank and beclomethasone dipropionate concentrations were determined by HPLC. The values of beclomethasone dipropionate concentrations at the upper part of the emulsifying tank were calculated in the case where beclomethasone dipropionate concentrations were 100% at the lower part of the emulsifying tank.

Successively, the beclomethasone dipropionate concentrations of the aqueous pharmaceutical compositions recovered from the emulsifying tank were determined by HPLC and the recovery s of beclomethasone dipropionate were obtained based on the theoretical values of the beclomethasone dipropionate concentrations calculated form the charged amounts of the compositions. Table 2 shows these values.

### Comparative example 2

Aqueous pharmaceutical compositions comprising the following components were prepared in the scale of 300 ml by the homomixer treatment. The homomixer treatments were carried out at 6,000 rpm for 30 minutes.

| (Composition 8) | |
|---|---|
| Beclomethasone dipropionate | 0.1% w/w |
| Microcrystalline cellulose carmellose sodium | 1.7% w/w |
| Polysorbate 80 | 0.1% w/w |

| (Composition 9) | |
|---|---|
| Beclomethasone dipropionate | 0.1% w/w |
| Microcrystalline cellulose carmellose sodium | 1.7% w/w |
| Polysorbate 60 | 0.1% w/w |

Immediately after the compositions 8 and 9 were treated by the homomixer, the aqueous pharmaceutical compositions were collected from the upper part and the lower part of the emulsifying tank and beclomethasone dipropionate concentrations were determined by HPLC. Further the values of beclomethasone dipropionate concentrations at the upper part of the emulsifying tank were calculated in the case where beclomethasone dipropionate concentrations were 100% at the lower part of the emulsifying tank.

Successively, the beclomethasone dipropionate concentrations of the aqueous pharmaceutical composition recovered from the emulsifying tank were determined by HPLC and the recoveries of beclomethasone dipropionate were obtained based on the theoretical values of the beclomethasone dipropionate concentrations calculated form the charged amounts of the compositions. These values are shown by table 2.

**Table 2**

| | Preparation | Beclomethasone dipropionate concentration (%) immediately after treatment | | Recovery rate (%) |
|---|---|---|---|---|
| | | Upper part of emulsifying tank | Lower part of emulsifying tank | |
| Example 2 | Composition 3 | 162.4 | 100.0 | 97.4 |
| | Composition 4 | 103.4 | 100.0 | 98.0 |
| | Composition 5 | 100.4 | 100.0 | 99.7 |
| | Composition 6 | 99.2 | 100.0 | 101.5 |
| | Composition 7 | 101.2 | 100.0 | 100.2 |
| Comparative example 2 | Composition 8 | 116.5 | 100.0 | 97.9 |
| | Composition 9 | 180.7 | 100.0 | 84.2 |

### Explanation of table 2

The compositions 4 to 7 containing 0.1 to 5% of hydroxypropylmethylcellulose had uniform beclomethasone dipropionate concentrations in the emulsifying tank immediately after the homomixer treatment and approximately 100% recoveries. The composition 3 having a beclomethasone dipropionate concentration of 0.01% was reduced in uniformity in the emulsifying tank and in recovery. On the other hand, the composition 8 containing 0.1% of polysorbate 80 and the composition 9 containing 0.1% of polysorbate 60 had extremely reduced uniformity in spite of containing 0.1% of the wetting agent and had recoveries much lower than those of the compositions containing hydroxypropylmethylcellulose.

These results prove that the variation of concentration of the water-insoluble and/or low water-soluble medicament except ciclesonide during the production and the reduction in recoveries are avoided by making the composition containes hydroxypropylmethylcellulose.

## Claims

1. An aqueous pharmaceutical composition containing one or more water-insoluble and/or low water-soluble medicament except ciclesonide and hydroxypropylmethylcellulose, wherein the one or more water-insoluble and/or low water-soluble medicament except ciclesonide are dispersed as solid particles in the aqueous pharmaceutical composition.

2. The aqueous pharmaceutical composition according to claim 1 wherein the hydroxypropylmethylcellulose concentration is from 0.01% w/w to 30% w/w.

3. The aqueous pharmaceutical composition according to claim 1 wherein the hydroxypropylmethylcellulose concentration is from 0.01% w/w to 5% w/w.

4. The aqueous pharmaceutical composition according to claim 1 wherein the hydroxypropylmethylcellulose concentration is from 0.01% w/w to 1% w/w.

5. The aqueous pharmaceutical composition according to claim 1 wherein the hydroxypropylmethylcellulose concentration is from 0.01% w/w to 0.5% w/w.

6. The aqueous pharmaceutical composition according to any one claim of claim1 to claim 5 wherein the aqueous pharmaceutical composition additionally contains one or more water-insoluble and/or low water-soluble substance.

7. The aqueous pharmaceutical composition according to claim 6 wherein the one or more water-insoluble and/or low water-soluble substance is cellulose.

8. The aqueous pharmaceutical composition according to claim 7 wherein the cellulose is microcrystalline cellulose.

9. The aqueous pharmaceutical composition according to any one claim of claim 1 to claim 8 wherein the aqueous pharmaceutical composition additionally contains a water-soluble polymer substance.

10. The aqueous pharmaceutical composition according to claim 9 wherein the water-soluble polymer substance is one or more selected from the group consisting of polyethylene glycol, propylene glycol alginate, pectin, low methoxyl pectin, guar gum, gum arabic, carrageenan, methylcellulose, carboxymethylcellulose sodium, xanthan gum and hydroxypropylcellulose.

11. The aqueous pharmaceutical composition according to claim 9 wherein the water-soluble polymer substance is carboxymethylcellulose sodium.

12. The aqueous pharmaceutical composition according to claim 9 wherein the water-soluble polymer substance is polyethylene glycol.

13. The aqueous pharmaceutical composition according to claim 9 wherein the water-soluble polymer substance is hydroxypropylcellulose.

14. The aqueous pharmaceutical composition according to any one claim of claim 1 to claim 13 wherein the combination of the water-insoluble and the water-soluble polymer substance is microcrystalline cellulose carmellose sodium.

15. The aqueous pharmaceutical composition according to any one claim of claim 1 to claim 14 wherein the medicament is steroid.
